# FASCICULE DE BREVET EUROPEEN

(11) **EP 1 467 703 B1**
(45) Date de publication et mention de la délivrance du brevet: **11.07.2012**
(21) Numéro de dépôt: 03712258.7
(22) Date de dépôt: 17.01.2003
(51) Int. Cl.: A61K 8/02, A61Q 19/00, A61Q 19/08, A45D 34/00

(54) **PROCEDE DE TRAITEMENT NOTAMMENT COSMETIQUE D'UNE ZONE DE LA PEAU D'UN SUJET HUMAIN PAR VOIE TRANS-EPIDERMIQUE, PAR PROJECTION D'UN LIQUIDE SOUS PRESSION**
BEHANDLUNGSVERFAHREN, INSBESONDERE KOSMETISCHER ART, EINER HAUTZONE EINES MENSCHLICHEN WESENS ÜBER DEN TRANSEPIDERMISCHEN WEG MITTELS AUFTRAGENS EINER FLÜSSIGKEIT UNTER DRUCK
METHOD FOR TREATMENT OF A REGION OF THE SKIN OF A HUMAN SUBJECT, IN PARTICULAR COSMETIC TREATMENT BY THE TRANS-EPIDERMAL ROUTE USING PROJECTION OF A LIQUID UNDER PRESSURE

(30) Priorité: 21.01.2002 FR 0200701
(43) Date de publication de la demande: 20.10.2004
(73) Titulaire: Société de Promotion et Diffusion d'Equipment Médical Medicamat, 92240 Malakoff (FR)
(72) Inventeur: DEVIDAL, Jean-Pierre, F-75005 Paris (FR); MICHAUD, Jean-Pierre, F-92140 Clamart (FR); CORRE, Alain, F-75011 Paris (FR)
(74) Mandataire: Novagraaf Technologies
(86) Numéro de dépôt international: PCT/FR2003/000153
(87) Numéro de publication internationale: WO 2003/061617

(56) Documents cités:
- EP-A- 1 110 570
- DE-A- 19 606 433

## Description

L'invention concerne un procédé et un dispositif de traitement notamment cosmétique d'une zone de la peau d'un sujet humain par voie trans-épidermique.

On connaît déjà des méthodes de rajeunissement de la peau telles que des méthodes au laser, des peelings chimiques, des techniques de mésothérapie, des injections de collagène ou d'autres produits, résorbables ou pas, ou des méthodes d'abrasion de la peau par projection de poudres abrasives.

Le document DE 196 06 433 décrit un procédé pour déposer des substances, en particulier pharmaceutiques ou cosmétiques, sous forme solide, liquide ou gazeuse sur une zone de la peau à traiter, par un dispositif d'injection sous pression. L'injection sous pression consiste ici à déposer des substances au travers de l'épiderme, en appliquant une pression déterminée sur la surface de la peau.

Le document EP 110 570 décrit un dispositif d'injection hypodermique pour l'administration sous cutanée, sans aiguille, d'un produit tel qu'un médicament, le produit étant propulsée sous pression à travers la peau du patient.

Toutefois, ces méthodes présentent plusieurs inconvénients : elles sont en effet susceptibles de provoquer des douleurs et/ou des effets secondaires.

En outre, elles sont le plus souvent socialement invalidantes pour les patients, car la peau, ayant été fortement sollicitée, ne retrouve son aspect normal qu'après un laps de temps important

Pour pallier ces inconvénients, l'invention propose un procédé de traitement notamment cosmétique d'une zone de la peau d'un sujet humain par voie trans-épidermique, destiné notamment à permettre le rajeunissement de la peau et/ou l'amélioration des imperfections de la peau.

La mise en oeuvre de ce procédé permet de faire pénétrer des substances actives au travers de l'épiderme, sans provoquer d'invalidation importante: Ainsi, une fois le traitement terminé, la peau retrouve son aspect normal dans un laps de temps très court, allant de quelques minutes à quelques heures, et les patients peuvent par conséquent reprendre très rapidement leurs activités habituelles.

En outre, cette méthode ne nécessite aucune anesthésie, et ne provoque pas de douleurs chez le patient. Elle permet de faire pénétrer en profondeur des produits actifs, destinés à améliorer notamment l'aspect de la peau, par exemple l'hydratation.

A cet effet, et selon un premier aspect, l'invention propose un dispositif pour le traitement d'une zone de la peau d'un sujet humain par voie trans-épidermique, conforme à la revendication 1.

Selon un deuxieme aspect, l'invention concerne un procédé de traitement cosmétique non thérapeutique d'une zone de la peau conforme à la revendication 7.

D'autres objets et avantages de l'invention apparaîtront au cours de la description qui suit, faite en référence aux dessins annexés dans lesquels :
- La figure 1 est une vue schématique de face d'un dispositif pour la mise en oeuvre du procédé selon l'invention ;
- La figure 2 est une vue schématique des éléments formant le dispositif de la figure 1, selon un premier mode de réalisation dans lequel le dispositif fonctionne en circuit ouvert;
- La figure 3 est une vue schématique des éléments formant le dispositif de la figure 1, selon un deuxième mode de réalisation dans lequel le dispositif fonctionne en circuit fermé ;
- La figure 4 est une vue schématique des moyens de mise en pression du dispositif de la figure 1 selon un mode de réalisation ;
- La figure 5 est une vue en coupe d'une pièce à main faisant partie du dispositif de la figure 1, selon un premier mode de réalisation, comprenant une buse de projection d'un liquide sous pression ;
- Les figures 6a et 6b sont respectivement des vues en coupe et de dessus d'une pièce à main faisant partie du dispositif de la figure 1, selon un deuxième mode de réalisation ;
- Les figures 7a, 7b et 7c sont respectivement des vues partielles en coupe de la pièce à main de la figure 5, montrant la forme du jet de liquide projeté sur la peau, selon trois variantes ;
- La figure 8 est une vue schématique des éléments formant le dispositif de la figure 1, selon une variante du deuxième mode de réalisation dans lequel le dispositif fonctionne en circuit fermé.

Le procédé selon l'invention est destiné à permettre la pénétration de substances actives au travers de l'épiderme.

Selon l'invention, ce procédé comprend l'étape consistant à projeter par une buse un liquide sous pression sur une zone à traiter.

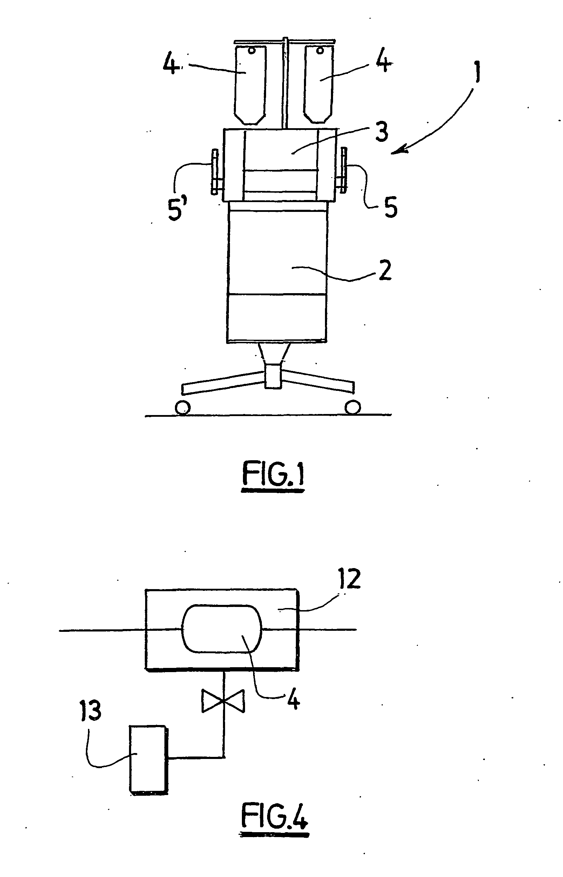

Ce procédé est mis en oeuvre par un dispositif 1 tel que celui représenté sur la figure 1. Ce dispositif 1 comprend un logement 2 dans lequel des moyens de mise en pression d'un liquide et des moyens d'aspiration dudit liquide sont disposés, un clavier de commande 3, des poches 4 destinées à recevoir un ou plusieurs liquides, ainsi qu'une pièce à main 5 permettant à un opérateur de mettre en oeuvre ledit procédé.

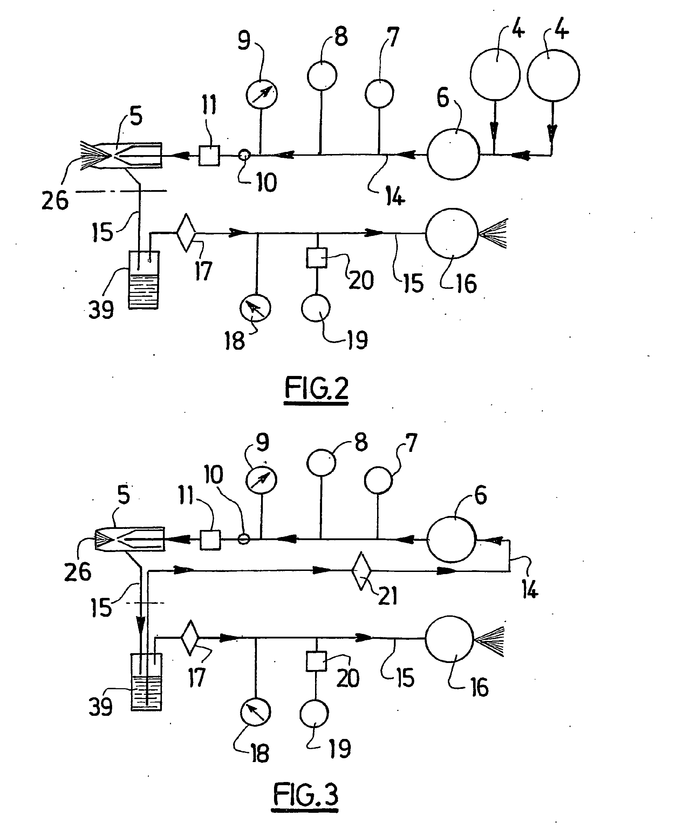

Selon un mode de réalisation, représenté sur les figures 2 et 3, les moyens de mise en pression du liquide comprennent une pompe 6 qui peut être une pompe à piston ou à membrane, apte à conférer au liquide une pression pouvant varier entre 5 et 70 bars. Les moyens de mise en pression sont associés à des moyens de contrôle et de mesure de la pression, comprenant, dans le sens de circulation du liquide, un amortisseur 7, un pressostat 8, un manomètre 9, un décompresseur 10 et une électrovanne 11.

Selon un autre mode de réalisation, représenté sur la figure 4, la mise sous pression du liquide peut être effectuée par une enceinte 12, dans laquelle la poche souple 4 contenant le liquide est placée, ladite enceinte 12 étant mise sous pression par exemple par une bouteille d'air ou d'azote 13, ou par un surpresseur.

Ces moyens de mise en pression permettent d'amener le liquide sous pression par un conduit 14 jusqu'à la pièce à main 5, grâce à laquelle ledit liquide est projeté sur une zone de la peau.

La pièce à main 5 sera décrite plus loin de façon détaillée.

Après avoir été projeté sur la peau, le liquide est aspiré à travers un conduit 15. Les moyens d'aspiration comprennent une pompe à vide 16, associée à des moyens de filtration 17, ainsi qu'à des moyens de contrôle et de mesure du vide comprenant un vacuomètre 18, un vacuostat 19 et une électrovanne 20.

La figure 2 représente un premier mode de réalisation du dispositif dans lequel celui-ci fonctionne en circuit ouvert. Dans ce mode de réalisation, le liquide est jeté après son aspiration dans un bocal 39 prévu à cet effet.

Selon un deuxième mode de réalisation, représenté sur la figure 3, le dispositif fonctionne en circuit fermé. A cet effet, après aspiration du liquide, celui-ci est remis en circulation en passant à travers un filtre 21, de sorte à retenir les impuretés et les déchets cutanés contenus dans le liquide, puis le liquide est recyclé en passant de nouveau dans le circuit. Cet arrangement présente l'avantage, pour un même sujet, d'utiliser un volume de solution moindre.

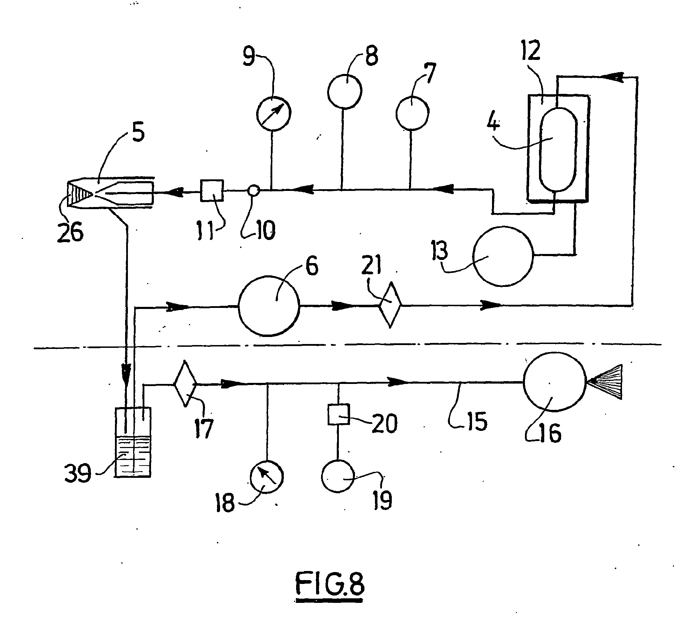

Selon une variante du deuxième mode de réalisation (figure 8), cet arrangement est mis en oeuvre lorsque le circuit comprend une enveloppe stérile mise en pression dans une enceinte 12, à travers laquelle passe le liquide, de sorte à éviter tout risque de contamination. Dans cette variante, tous les composants en contact avec le liquide sont adaptés à ces conditions de stérilisation.

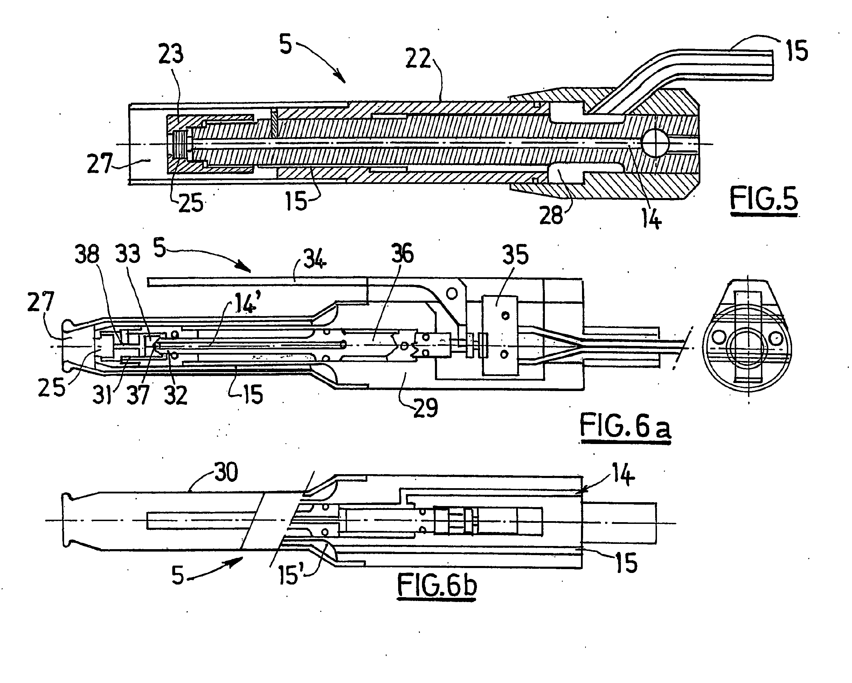

On décrit à présent la pièce à main 5, en relation avec les figures 5 et 6.

Cette pièce à main 5 est traversée par le conduit 14. Elle est constituée d'un corps 22 dont le matériau peut supporter le passage en autoclave en vue de sa stérilisation.

La pièce à main 5 est munie d'une vanne marche/arrêt, non représentée, destinée à contrôler la projection du liquide. En variante, cette vanne peut être disposée sur le corps du dispositif 1, mais cet arrangement présente l'inconvénient de conduire à un écoulement résiduel du liquide restant dans le conduit 14.

La pièce à main 5 est munie d'un embout 30 dévissable ou détachable destiné à obtenir un vacuum sur la peau, ledit embout 30 étant de forme arrondie, de sorte à ne pas endommager la surface de la peau sur laquelle il est appliqué.

La pièce à main 5 comprend en outre un porte-buse 23 démontable ou détachable, sur lequel est montée une buse de projection 25, également démontable, et stérilisable, destinée à projeter le liquide sous pression sur la zone à traiter. Le liquide arrive jusqu'à cette buse de projection 25 via le conduit 14.

Les buses de projection 25 utilisées sont constituées d'un matériau de dureté élevée, tel que du carbure de tungstène.

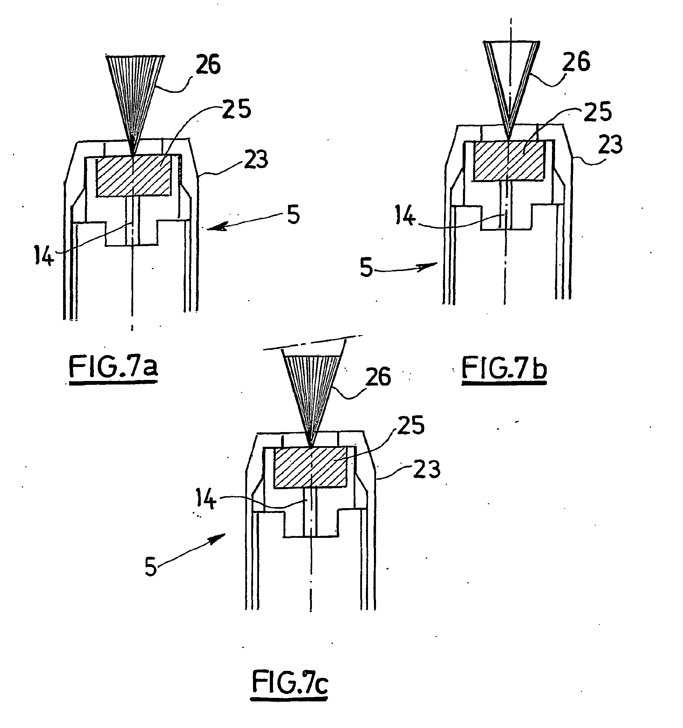

Elles permettent, selon leur géométrie, de former des jets de liquide de différentes formes, de différentes largeurs, ou de différentes distances focales. Trois variantes sont représentées sur les figures 7a, 7b et 7c, qui montrent des jets 26 dont la forme est respectivement de type pinceau triangulaire, ou cône plein (figure 7a), de type cône creux (figure 7b) et de type jet plat (figure 7c).

La pièce à main 5 comprend également une chambre d'aspiration annulaire 27 reliée par le conduit 15 à un collecteur 28, de sorte à permettre l'aspiration du liquide, après sa projection sur la peau.

Dans le mode de réalisation représenté sur la figure 6, la pièce à main 5 comprend un corps 29 et un embout 30 monté sur le corps 29 dont l'extrémité ouverte enveloppe la buse de projection 25.

En position de repos de la pièce à main 5, le liquide sous pression est arrêté par le contact de l'extrémité 32 du piston 36 sur la pièce 33.

Cette pièce à main comprend en outre des moyens de commande comprenant un levier de commande 34, un contacteur électrique 35 et un piston 36. Ces moyens de commande permettent, lorsqu'un utilisateur exerce un appui sur le levier 34, d'éloigner l'extrémité 32 de la pièce 33, de telle sorte que l'orifice 37 du conduit 14' libère le liquide qui remplit alors la chambre de distribution 31 et, par l'intermédiaire du conduit 38, alimente la buse de projection 25.

Lesdits moyens de commande sont également agencés pour contrôler le fonctionnement des moyens d'aspiration du liquide après sa projection sur la peau. Ces moyens d'aspiration comprennent une section annulaire 15' et un conduit cylindrique 15.

Le liquide projeté comporte typiquement de l'eau, ou du sérum physiologique éventuellement mélangé avec un ou plusieurs additifs à usage cosmétique, thérapeutique ou anesthésique.

On décrit à présent un mode de réalisation du procédé selon l'invention.

Afin de faciliter la pénétration des substances actives, le procédé de traitement de la peau par voie trans-épidermique peut être précédé d'une étape d'abrasion du stratum corneum et éventuellement des couches supérieures de l'épiderme au moyen de produits abrasifs tels que des noyaux concassés, de l'hydroxyde d'alumine, des microbilles de verre ou de céramique, ou tout autre produit abrasif présentant par exemple une granulométrie de l'ordre de 100 à 125 microns.

Cette étape est mise en oeuvre par une pièce à main 5' (figure 1), agencée pour permettre l'abrasion de la peau par des produits abrasifs. Ainsi, il est possible d'utiliser des quantités d'additifs faiblement dosées dans l'étape de projection du liquide.

L'abrasion du stratum comeum et la pression conférée ensuite au jet de liquide permettent de faire pénétrer profondément dans l'épiderme des produits qui, sous forme cosmétique, ne pénètrent habituellement que les couches supérieures de l'épiderme.

La solution destinée à être projetée sur la peau peut ensuite être préparée en injectant un ou plusieurs additifs, au moyen d'une seringue et d'une aiguille, dans la poche souple 4 contenant de l'eau stérilisée, ou du sérum physiologique.

Il est également possible d'utiliser des solutions préparées extemporanément ou des produits existant dans le commerce.

Puis, dès que cette injection est réalisée, l'étape suivante du procédé est mise en oeuvre. Ainsi, la solution demeure stable, et le ou les additifs demeurent solubles en solution.

L'étape suivante consiste à former un vacuum sur la peau, puis les moyens de mise en pression sont activés de sorte à permettre la projection sous pression de la solution sur la peau du sujet, au moyen de la buse de projection 25.

Un dispositif de sécurité permet de s'assurer que les moyens de mise en pression ne soient mis en fonctionnement que lorsque ledit vacuum est formé sur la peau, de sorte à éviter toute projection intempestive de solution.

La projection de ladite solution est réalisée à une pression telle qu'il n'y ait pas de risque d'effraction cutanée, tout en imposant une pression maximale pour une pénétration efficace de ladite solution.

Dans un exemple particulier, les pressions utilisées sont comprises entre 10 et 25 bars, de sorte à permettre une pénétration efficace de la solution dans la peau.

Dans un exemple particulier, les débits utilisés sont compris entre 100 et 250 ml/minute. Lorsque la solution est projetée sur le visage du sujet, la durée de cette étape est de l'ordre de quelques minutes, le volume de solution utilisé étant typiquement de l'ordre de 1 à 2 litres.

Au fur et à mesure de la projection de ladite solution, celle-ci est aspirée dans la chambre d'aspiration 27, en vue de son évacuation par le conduit 15. La solution est alors stockée dans un bocal 39 (figures 2 et 3), de sorte à être jetée après son usage (mode de fonctionnement en circuit ouvert), ou filtrée de sorte à être recyclée (mode de fonctionnement en circuit fermé).

Les additifs utilisés peuvent être des oligo-éléments (zinc, silicium, sélénium...), des vitamines (A, E, C, K...), des acides tels que des acides de fruits (canne à sucre, pamplemousse...), les acides trichloracétique, kojique, hyaluronique, salicylique..., des sels minéraux, des extraits de plantes, ou tout autre additif choisi selon ses propriétés spécifiques. Ces propriétés peuvent conduire, par exemple, à des fonctions hydratantes, anti-radicaux libres, régénérantes, restructurantes, exfoliantes, adoucissantes, nourrissantes, anti-inflammatoires, cicatrisantes, blanchissantes ou dépigmentantes, cette liste n'étant bien entendu pas exhaustive.

D'autres additifs peuvent également être utilisés, tels que des anesthésiques.

A titre d'exemple, les additifs suivants peuvent être utilisés :
- huile de rosier muscat, huile de pépin de raisin, pour leurs propriétés régénératrices et restructurantes de la peau ;
- extrait de prêle, pour son action régénérante et raffermissante ;
- tocophérol, la vitamine E ayant des propriétés anti-radicalaires ;
- α-hydroxyacide (AHA) de canne à sucre, pour ses propriétés exfoliantes ;
- extrait de vigne rouge, pour ses propriétés anti-rougeurs et décongestionnantes ;
- extrait d'Arnica Montana, pour ses propriétés anti-couperose, antiseptique et astringente ;
- acide hyaluronique, pour son action hydratante ;
- silicium organique ;
- thé vert, pour ses propriétés anti-radicalaire et astringentes ;
- extrait de racine de carotte, riche en vitamines B1, C et E ;
- extrait de busserole, qui contient de l'arbutine, connue pour être un inhibiteur de la mélanine ;
- molécule HPS3^{®}, obtenue à partir d'un extrait purifié de l'algue Padina Pavonica, et capable de stimuler la synthèse des glucosaminoglycanes, essentiels dans l'hydratation de la peau ;
- lidocaïne, xylocaïne.

## Revendications

1. Dispositif pour le traitement d'une zone de la peau d'un sujet humain par voie trans-épidermique, comprenant une pièce à main (5), une buse de projection (25) montée sur ladite pièce (5), des moyens de mise en pression d'un liquide, et au moins un conduit (14) disposé entre les moyens de mise en pression et la buse de projection (25), ledit dispositif étant **caractérisé en ce qu'**il comprend une chambre d'aspiration annulaire (27).

2. Dispositif selon la revendication 1, **caractérisé par le fait que** les moyens de mise en pression comprennent une pompe (6).

3. Dispositif selon la revendication 2, **caractérisé par le fait que** les moyens de mise en pression comprennent une poche (4) apte à recevoir ledit liquide et une enceinte (12) apte à recevoir ladite poche (4).

4. Dispositif selon l'une des revendications 1 à 3, **caractérisé par le fait qu'**il comprend un embout (30) dévissable ou détachable délimitant ladite chambre (27) ouverte vers l'extérieur, et des moyens pour faire le vide dans ladite chambre (27).

5. Dispositif selon l'une quelconque des revendications 1 à 4, **caractérisé par le fait qu'**il comprend des moyens de filtration (21).

6. Dispositif selon l'une des revendications 1 à 5, **caractérisé en ce que** la pièce à main comprend des moyens de commande agencés pour, en position de repos de ladite pièce à main, arrêter le liquide sous pression par le contact de l'extrémité (32) d'un piston (36) sur une pièce (33) et, en position d'utilisation, éloigner ladite extrémité (32) de la pièce (33), de sorte à libérer le liquide pour alimenter la buse de projection (25).

7. Procédé de traitement cosmétique non thérapeutique d'une zone de la peau d'un sujet humain par voie trans-épidermique au moyen d'un dispositif de traitement selon l'une des revendications 1 à 6, comprenant une étape consistant à projeter un liquide sous une pression comprise entre 5 et 70 bars sur la zone à traiter, au moyen d'une buse de projection (25), et une étape consistant à aspirer le liquide projeté en vue de l'évacuer.

8. Procédé selon la revendication 7, **caractérisé par le fait que** le liquide comporte de l'eau ou du sérum physiologique.

9. Procédé selon la revendication 8, **caractérisé par le fait que** le liquide comprend au moins un additif.

10. Procédé selon l'une quelconque des revendications 7 à 9, **caractérisé par le fait que** l'étape consistant à projeter un liquide sur la zone à traiter comprend la projection à une pression telle qu'il n'y ait pas de risque d'effraction cutanée de ladite zone, tout en imposant une pression maximale pour une pénétration efficace dudit liquide.

11. Procédé selon la revendication 10, **caractérisé en ce que** la pression est comprise entre 10 et 25 bars.

12. Procédé selon la revendication 7, **caractérisé en ce que** l'étape d'aspiration est effectuée à l'aide d'une chambre d'aspiration annulaire (27).

13. Procédé selon l'une quelconque des revendications 7 à 12, **caractérisé en ce qu'**il comprend les étapes consistant à filtrer le liquide et à le recycler.

14. Procédé selon l'une quelconque des revendications 7 à 13, **caractérisé en ce que** l'étape consistant à projeter un liquide sous pression sur la zone à traiter, au moyen d'une buse de projection (25) est précédée d'une étape d'abrasion du stratum corneum.

## Claims

1. Device for transcutaneous treatment of an area of the skin of a human subject comprising a handpiece (5), a spray nozzle (25) mounted on said handpiece (5), means for pressurising a liquid, and at least one pipe (14) disposed between the pressurising means and the spray nozzle (25), said device being **characterised in that** it comprises an annular suction chamber (27).

2. Device according to claim 1 **characterised in that** the pressurising means comprise a pump (6).

3. Device according to claim 2 **characterised in that** the pressurising means comprise a sachet (4) adapted to receive said liquid and an enclosure (12) adapted to receive said sachet (4).

4. Device according to any one of claims 1 to 3 **characterised in that** it comprises a tip (30) adapted to be unscrewed or detached delimiting said chamber (27) open to the exterior and means for producing a vacuum in said chamber (27).

5. Device according to any one of claims 1 to 4 **characterised in that** it comprises filter means (21).

6. Device according to any one of claims 1 to 5 **characterised in that** the handpiece comprises control means adapted, in a rest position of said handpiece, to stop the pressurised liquid by contact of the end (32) of a piston (36) on a part (33) and, in a position of use, to move away said end (32) of the part (33) so as to release the liquid to feed the spray nozzle (25).

7. Method for non-therapeutic transcutaneous treatment of an area of the skin of a human subject by means of a treatment device according to any one of claims 1 to 6, comprising a step consisting in spraying a liquid at a pressure in the range 5 bar to 70 bar onto the area to be treated by means of a spray nozzle (25) and a step consisting in aspirating the sprayed liquid in order to evacuate it.

8. Method according to claim 7 **characterised in that** the liquid contains water or physiological serum.

9. Method according to claim 8 **characterised in that** the liquid contains at least one additive.

10. Method according to any one of claims 7 to 9 **characterised in that** the step consisting in spraying a liquid onto the area to be treated comprises spraying at a pressure such that there is no risk of cutaneous breakage in said area whilst imposing a maximum pressure for efficacious penetration of said liquid.

11. Method according to claim 10 **characterised in that** the pressure is in the range 10 bar to 25 bar.

12. Method according to claim 7 **characterised in that** the aspiration step is effected using an annular aspiration chamber (27).

13. Method according to any one of claims 7 to 12 **characterised in that** it comprises steps consisting in filtering and recycling the liquid.

14. Method according to any one of claims 7 to 13 **characterised in that** the step consisting in spraying a pressurised liquid onto the area to be treated by means of a spray nozzle (25) is preceded by a step of abrasion of the stratum corneum.

## Patentansprüche

1. Vorrichtung zur transdermalen Behandlung eines Bereichs der menschlichen Haut umfassend ein Handstück (5), eine an besagtem Teil (5) befestigte Druckdüse (25), Mittel zum Unterdrucksetzen einer Flüssigkeit, und mindestens eine zwischen den Mitteln zum Unterdrucksetzen und der Druckdüse (25) angebrachte Leitung (14), wobei besagte Vorrichtung **dadurch gekennzeichnet ist, dass** sie eine ringförmige Saugkammer (27) umfasst.

2. Vorrichtung nach Anspruch 1, **dadurch gekennzeichnet, dass** die Mittel zum Unterdrucksetzen eine Pumpe (6) umfassen.

3. Vorrichtung nach Anspruch 2, **dadurch gekennzeichnet, dass** die Mittel zum Unterdrucksetzen der Flüssigkeit einen Beutel (4), der besagte Flüssigkeit aufnehmen kann, und einen zur Aufnahme des Beutels (4) geeigneten Behälter (12) umfassen.

4. Vorrichtung nach einem der Ansprüche 1 bis 3, **dadurch gekennzeichnet, dass** sie einen schraubbaren oder entfernbaren Verschluss (30), der die Kammer (27) begrenzt und nach Außen geöffnet ist, sowie Mittel zum Leeren der Kammer (27) umfassen.

5. Vorrichtung nach einem der Ansprüche 1 bis 4, **dadurch gekennzeichnet, dass** sie Mittel zum Filtern umfasst (21).

6. Vorrichtung nach einem der Ansprüche 1 bis 5, **dadurch gekennzeichnet, dass** das Handstück derart angebrachte Steuermittel umfasst, dass in der Ruhestellung des Handstücks die unter Druck stehende Flüssigkeit durch Kontakt des Endes (32) eines Kolbens (36) mit einem Teil (33) zurückgehalten wird, während in der Benutzungsstellung, das Entfernen des Endes (32) von dem Teil (33), die Flüssigkeit zur Versorgung der Druckdüse (25) freisetzt.

7. Verfahren zur kosmetischen nicht therapeutischen transdermalen Behandlung eines Bereichs der menschlichen Haut mit Hilfe einer Behandlungs-Vorrichtung nach einem der Ansprüche 1 bis 6, das einen Schritt des Sprühens einer Flüssigkeit auf den zu behandelnden Bereich mit einem Druck von 5 bis 70 bar mittels einer Druckdüse (25) und einen Schritt der Ansaugung der gesprühten Flüssigkeit zu ihrer Entfernung umfasst.

8. Vorrichtung nach Anspruch 7, **dadurch gekennzeichnet, dass** die Flüssigkeit Wasser oder physiologische Kochsalzlösung umfasst.

9. Vorrichtung nach Anspruch 8, **dadurch gekennzeichnet, dass** die Flüssigkeit mindestens einen Zusatzstoff umfasst.

10. Verfahren nach einem der Ansprüche 7 bis 9, **dadurch gekennzeichnet, dass** der Schritt, der daraus besteht, eine Flüssigkeit auf den zu behandelnden Bereich zu sprühen, das Sprühen unter solch einem Druck umfasst, dass kein Risiko des Durchbrechens der Haut in besagtem Bereich besteht, wobei gleichzeitig ein maximaler Druck für ein wirksames Eindringen der Flüssigkeit in die Haut vorgegeben wird.

11. Verfahren nach Anspruch 10, **dadurch gekennzeichnet, dass** der Druck zwischen 10 und 25 bar liegt.

12. Verfahren nach Anspruch 7, **dadurch gekennzeichnet, dass** der Ansaugschritt mit Hilfe einer ringförmigen Saugkammer (27) durchgeführt wird.

13. Verfahren nach einem der Ansprüche 7 bis 12, **dadurch gekennzeichnet, dass** es Schritte zum Filtern und zur Zurückführung der Flüssigkeit umfasst.

14. Verfahren nach einem der Ansprüche 7 bis 13, **dadurch gekennzeichnet, dass** der Schritt, der daraus besteht, Flüssigkeit unter Druck auf den zu behandelnden Bereich mit Hilfe einer Spraydüse (25) zu sprühen, auf einen Abrasionsschritt der Hornschicht folgt.
